(19) Europäisches Patentamt / European Patent Office / Office européen des brevets

(11) **EP 4 067 353 A1**

(12) **EUROPEAN PATENT APPLICATION**


(43) Date of publication:
**05.10.2022 Bulletin 2022/40**

(21) Application number: **22166176.2**

(22) Date of filing: **31.03.2022**

(51) International Patent Classification (IPC):
***C07D 409/06*** *(2006.01)* ***C09K 9/00*** *(2006.01)*
***C09K 11/00*** *(2006.01)*

(52) Cooperative Patent Classification (CPC):
**C07D 409/06; C09K 9/00; C09K 11/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021062097**
**24.02.2022 JP 2022026474**



(71) Applicant: **Hoya Lens Thailand Ltd.**
**Pathumthani 12130 (TH)**

(72) Inventors:
- **ARAI, Yuko**
**Tokyo, 160-8347 (JP)**
- **KOBAYASHI, Kei**
**Tokyo, 160-8347 (JP)**
- **NUSHI, Yusuke**
**Tokyo, 160-8347 (JP)**
- **HERON, Mark**
**Huddersfield, HD1 3DH (GB)**
- **GABBUTT, Christopher**
**Huddersfield, HD1 3DH (GB)**
- **CARVALHO COUTO DE AZEVEDO, Orlando Delfim**
**Huddersfield, HD1 3DH (GB)**

(74) Representative: **Beckmann, Claus**
**Kraus & Weisert**
**Patentanwälte PartGmbB**
**Thomas-Wimmer-Ring 15**
**80539 München (DE)**


(54) **PHOTOCHROMIC COMPOUND, PHOTOCHROMIC ARTICLE AND EYEGLASSES**


(57) The present application relates to photochromic compounds represented by the following general formula (1):

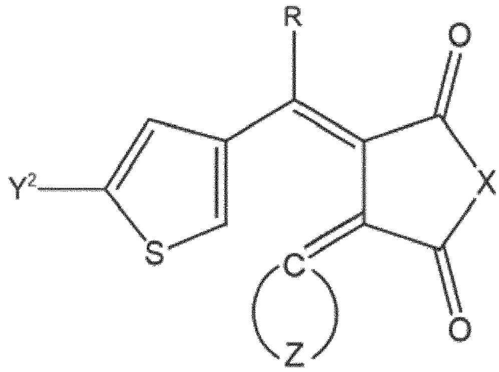

The application also relates to uses of said compounds in articles.


EP 4 067 353 A1

## Description

### TECHNICAL FIELD

The present invention relates to a photochromic compound, a photochromic article and eyeglasses.

### BACKGROUND

A photochromic compound is a compound having the property (photochromic property) of developing color under irradiation with light of the wavelength range having photoresponsive properties and fading the color under a non-irradiation state (for example, see PTL1). Examples of articles (photochromic articles) provided with the photochromic property by a photochromic compound may include optical articles such as spectacle lenses.

PTL1: US Patent No. 5,645,767

### SUMMARY OF THE INVENTION

PTL 1 discloses an indeno-fused naphthopyran-based compound as a photochromic compound. In contrast, while considering coexisting a photochromic compound with an oriented liquid crystal compound in a photochromic article in order to improve the performance of the photochromic article, the present inventors have revealed that the performance of a naphthopyran-based compound as a photochromic compound is easy to be degraded under the coexistence with a liquid crystal compound. The coexistence of a photochromic compound with an oriented liquid crystalline compound enables the molecular orientation direction of the photochromic compound to be aligned in a single direction (i.e., uniaxial orientation can be achieved) due to the oriented liquid crystalline compound, and the photochromic compound thereby is expected to develop dichroism. The "dichroism" means a nature that the color of transmitted light varies depending on the direction of propagation because a medium has anisotropy of the selective absorption for light. If a compound develops dichroism, light absorption becomes strong in a particular direction of molecules with respect to polarized light, and light absorption becomes small in a direction orthogonal to the above direction. If a photochromic compound can develop dichroism in a photochromic article, the photochromic article can show polarization performance in addition to photochromic performance.

One aspect of the present invention provides for a photochromic compound that shows less performance degradation under the coexistence with an oriented liquid crystalline compound.

As a result of intensive study, the present inventors have newly found that a fulgide compound represented by the following general formula (1) can show less performance degradation under the coexistence with an oriented liquid crystal compound.

That is, one aspect of the present invention relates to a photochromic compound represented by the following general formula (1).

General formula (1)

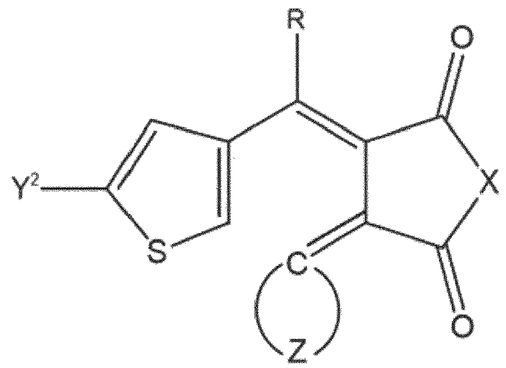

In the general formula (1),

X denotes an oxygen atom or a nitrogen atom unsubstituted or substituted by a substituent selected from the following $Y^1$ group:
$Y^1$ group: $-R^1$, $-A^1(B^1)_l(A^2)_m(B^2)_nR^2$, $-A^3A^4$, $-A^5R^3$
$R^1$ denotes a cyano group, or an alkyl group or an aryl group which may each be substituted,

$R^2$ denotes an alkyl group, a naphthyl group, or a naphthylalkyl group which may each be substituted,
$R^3$ denotes a halogen atom, a cyano group, or a nitro group,
$A^1$, $A^2$, $A^3$, and $A^5$ each independently denote an alkylene group, an alkylidene group, a cycloalkylene group, or an alkylcycloalkane-diyl group which may each be substituted,
$A^4$ denotes a naphthyl group which may be substituted,
$B^1$ and $B^2$ each independently denote any one of the divalent groups selected from the following group:

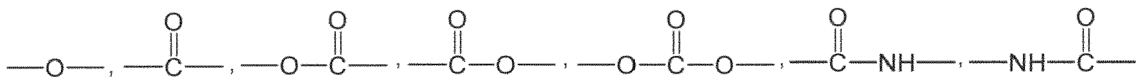

l, m, and n are each independently 0 or 1, provided that n is 0 if m is 0,
$Y^2$ denotes a hydrogen atom, or a substituent selected from the above $Y^1$ group,
R denotes a hydrogen atom, a trifluoromethyl group, or a cyclopropyl group which may be substituted,

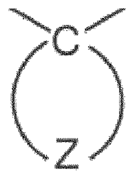

denotes a norbornylidene group, a bicyclo[3.3.1]nonylidene group, or an adamantylidene group which may each be substituted.

According to one aspect of the present invention, a photochromic compound that shows less performance degradation under the coexistence with an oriented liquid crystalline compound can be provided.

DESCRIPTION OF THE EMBODIMENTS

Photochromic Compound

The photochromic compound undergoes an excited state by receiving the irradiation of light such as sunlight and is structurally converted into a colored body, as one example. The structure after structural conversion via irradiation with light can be referred to as a "colored body". In contrast, the structure before light irradiation can be referred to as a "colorless body". The term "colorless" in the "colorless body" does not limitedly mean a complete colorless state and encompasses a state where the color is pale compared to the colored body. The structure of the general formula (1) is a structure of a colorless body. The photochromic compound represented by the general formula (1) has a colorless body with a ring-opened structure.
The photochromic compound represented by the general formula (1) is hereinafter described in more detail. In the present invention and the present description, the photochromic compound represented by the general formula (1) encompasses geometrical isomers of a structure indicated as the general formula (1).
In the general formula (1), X denotes an oxygen atom, or a nitrogen atom unsubstituted or substituted with a substituent selected from the $Y^1$ group described below.
$Y^1$ group: $-R^1$, $-A^1(B^1)_l(A^2)_m(B^2)_nR^2$, $-A^3A^4$, $-A^5R^3$
$R^1$ denotes a cyano group, an alkyl group which may be substituted, or an aryl group which may be substituted.
Examples of alkyl groups represented by $R^1$ may include a methyl group, an ethyl group, a propyl group, a n-, iso-, or ter-butyl group, a pentyl group, a hexyl group, an octyl group, a decyl group, and the like. Among these, an alkyl group with C (i.e., carbon number) 1-20 is preferable, and an alkyl group with C1-10 is more preferable.
Examples of aryl groups represented by $R^1$ may include an aryl group with C6-10 such as a phenyl group, a biphenyl group, a tolyl group, or a naphthyl group.
$R^2$ denotes an alkyl group which may be substituted, a naphthyl group which may be substituted, or a naphthylalkyl group which may be substituted.
The former description about the alkyl group represented by $R^1$ can be applied to the description about the alkyl group represented by $R^2$. The carbon number in an alkyl group represented by $R^2$ is not particularly limited and may preferably be 1 to 10.
Examples of the naphthylalkyl group represented by $R^2$ may include a naphthylmethyl group, a naphthylethyl group, a naphthylpropyl group, a naphthyl butyl group, or the like. The carbon number in the alkyl group of the naphthylalkyl

group is preferably 1 to 4.

$R^3$ denotes a halogen atom, a cyano group, or a nitro group. Examples of the halogen atom represented by $R^3$ include a fluorine atom, a chlorine atom, a bromine atom, or the like.

$A^1$, $A^2$, $A^3$, and $A^5$ each independently denote an alkylene group which may be substituted, an alkylidene group which may be substituted, a cycloalkylene group which may be substituted, or an alkylcycloalkane-diyl group which may be substituted. Specific examples of these may include alkylene groups with C1-10 such as a methylene group, an ethylene group, a propylene group, a butylene group, a trimethylene group, a tetramethylene group, or a 2,2-dimethyltrimethylene group; alkylidene groups with C2-10 such as an ethylidene group, a propylidene group, or an isopropylidene group; and cycloalkylene groups with C3-10 such as a cyclohexylene group; and alkylcycloalkane-diyl groups with C6-10 such as a 2-methylcyclohexane-a,1-diyl group represented by the following formula:

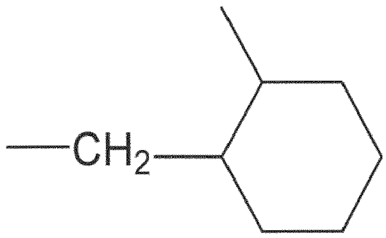
,

and a 4-methylcyclohexane-a,1-diyl group represented by the following formula:

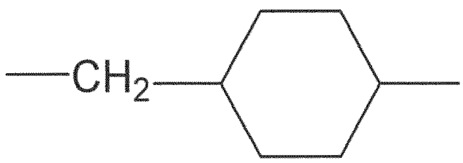
.

It is preferable that $A^1$, $A^2$, $A^3$, and $A^5$ are each independently an alkylene groups with C1-6, an alkylidene group with C2-6, a cycloalkylene group with C3-6, or an alkylcycloalkane-diyl group with C6-7.

$B^1$ and $B^2$ each independently denote any one of the divalent groups selected from the following group.

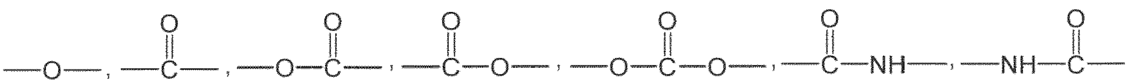

l, m, and n are each independently 0 or 1, provided that n is 0 if m is 0,

Each of the groups described above is unsubstituted in one embodiment and is substituted in other one embodiment. The substituent is not particularly limited. When a group has a substituent, the carbon number described above shall refer to the carbon number of the part which does not include a substituent. For example, the alkyl group may be substituted with one to three atoms and/or groups selected from the group consisting of halogen atoms, a cyano group, and a nitro group. The naphthyl group or the naphthylalkyl group may be substituted with one to three atoms and/or groups selected from the group consisting of halogen atoms, a cyano group, a nitro group, alkylamino groups with C1-3, alkyl groups with C1-3, or alkoxy groups with C1-3.

$A^4$ denotes a naphthyl group which may be substituted. The naphthyl group represented by $A^4$ is unsubstituted in one embodiment and is substituted in other one embodiment. The type of such a substituent is not particularly limited. For example, the naphthyl group represented by $A^4$ may be substituted with one to three atoms and/or groups selected from the group consisting of halogen atoms, a cyano group, and a nitro group, alkylamino groups with C1-3, alkyl groups with C1-3, and alkoxy groups with C1-3.

$Y^2$ denotes a hydrogen atom, or a substituent selected from the $Y^1$ group described above. $Y^2$ is preferably a hydrogen atom in one embodiment. In other one embodiment, $Y^2$ is preferably $-R^1$ in the $Y^1$ group, more preferably an aryl group which may be substituted, further preferably an aryl group which is substituted, and still more preferably a substituent represented by $-R^{11}-R^{12}$, wherein the $-R^{11}-$ is selected from the group consisting of the following group, and the $-R^{12}$ is $-(CH_2)_nCH_3$, where n is an integer within the range of 3-10.

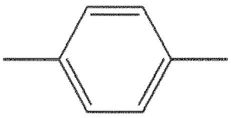 , 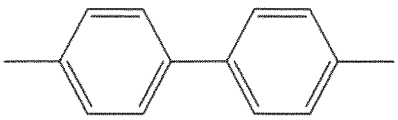 , 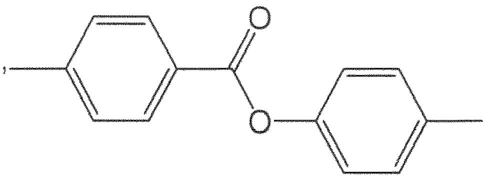

R denotes a hydrogen atom, a trifluoromethyl group, or a cyclopropyl group which may be substituted. The cyclopropyl group represented by R is unsubstituted in one embodiment and is substituted in other one embodiment. As the substituent thereof, a substituent selected from the $Y^1$ group described above may be exemplified. In one embodiment, R preferably denotes a cyclopropyl group which may be substituted and more preferably denotes an unsubstituted cyclopropyl group.

In the general formula (1),

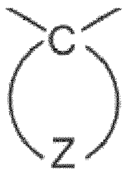

denotes a norbornylidene group which may be substituted, a bicyclo[3.3.1]nonylidene group which may be substituted, and an adamantylidene group which may be substituted.

As the norbornylidene group, the following 7-norbornylidene group is preferable.

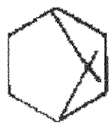

As the bicyclo[3.3.1]nonylidene group, the following bicyclo[3.3.1]9-nonylidene group is preferable.

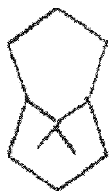

As the adamantylidene group, the following 2-adamantylidene group is preferable.

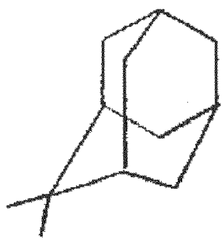

The above structures of the 7-norbornylidene group, the bicyclo[3.3.1]nonylidene group, and the 2-adamantylidene group are all structures with no substituent.

In the general formula (1), a norbornylidene group, a bicyclo[3.3.1]nonylidene group, and an adamantylidene group represented by

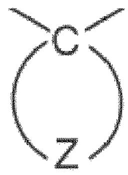

are all unsubstituted in one embodiment and are substituted in other one embodiment. When a group has substituents, the number of substituents may be one, or two or more. Examples of the substituent may include a hydroxy group; alkylamino groups with C1-4 such as a methylamino group and a diethylamino group; alkoxy group with C1-4 such as a methoxy group, an ethoxy group, and a tert-butoxy group; aralkoxy groups with C7-15 such as a benzyloxy group; aryloxy group with C6-14 such as a phenoxy group and 1-naphthoxy group; alkyl group with C1-4 such as a methyl group, an ethyl group, and a t-butyl group; halogen atoms such as a fluorine atom, a chlorine atom, and a bromine atom; a cyano group; a carboxy group; alkoxycarbonyl group with C2-10 such as an ethoxycarbonyl group; a halogen-substituted alkyl group with C1-2 such as a trifluoromethyl group; a nitro group; an aryl group with C6-10 such as a phenyl group and a toluyl group, aralkyl group with C7-9 such as a phenylethyl group and a phenylpropyl group, and the like. Preferable examples of these substituents may include a halogen atom, a hydroxy group, alkyl groups with C1-4, alkoxy groups with C1-4, alkoxycarbonyl groups with C2-10, aralkyl groups with C7-9, aryl groups with C6-10, or the like.

The photochromic compound represented by the general formula (1) may be used for constructing an article (photochromic article) with the photochromic property. The following compounds can be listed as specific examples of such a compound. However, the above photochromic compound is not limited to the listed compounds. The following structures are structures of colorless bodies. A geometric isomer of the compound 1 (E body) is a compound 2 (Z body). A geometric isomer of the compound 3 (E body) is a compound 4 (Z body).

(Compound 1)

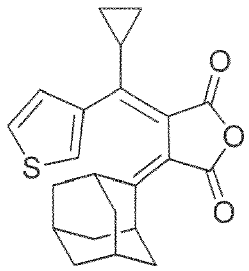

(Compound 2)

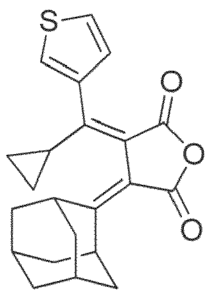

(Compound 3)

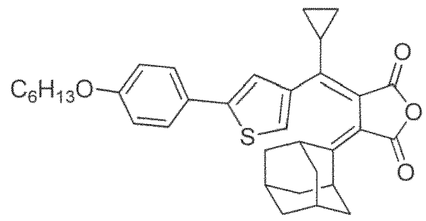

(Compound 4)

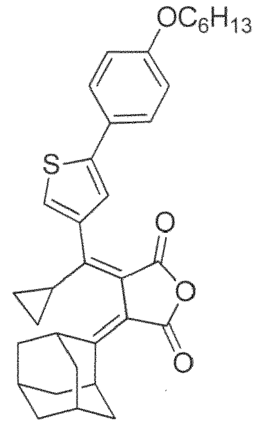

(Compound group 5)

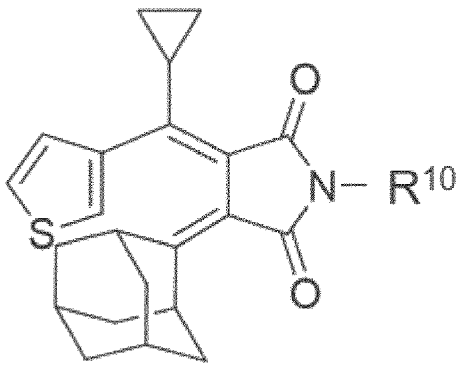

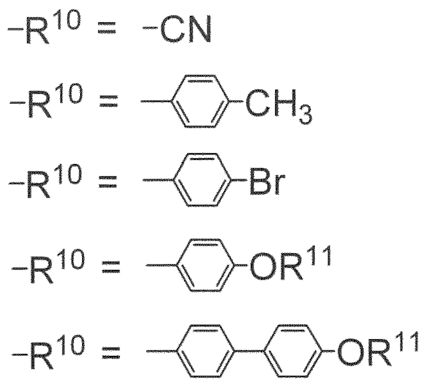

$R^{11}=(CH_2)_nCH_3$, $n=0\sim10$

The synthetic method of the photochromic compound represented by the general formula (1) is not particularly limited. For example, paragraphs [0068] to [0099] and the examples of Japanese Patent Application Publication No. H07-2824 can be referred to for the synthetic method thereof. Also, the identification method of the compound is not particularly limited. For example, paragraphs [0063] to [0066] of Japanese Patent Application Publication No. H07-2824 can be referred to for the identification method. The disclosure in the section of Examples, which will be described below, can also be referred to for the synthetic method thereof.

Photochromic Article

One aspect of the present invention relates to a photochromic article including the photochromic compound represented by the general formula (1).

In the present invention and the present description, a "photochromic article" shall mean an article including at least one photochromic compound. The photochromic compound represented by the general formula (1) may be included in the photochromic layer of a photochromic article and/or may be included in the substrate of a photochromic article. In an embodiment, such a photochromic article may include only one compound represented by the general formula (1), and in other one embodiment, such a photochromic article may include two or more compounds represented by the general formula (1). In one embodiment, the photochromic article may contain at least one compound represented by the general formula (1) and at least one other photochromic compound. Examples of the other photochromic compounds may include azobenzenes, spiropyrans, spirooxazines, naphthopyrans, indenonaphthopyrans, phenanthropyrans, hexaallylbisimidazoles, donor-acceptor Stenhouse adducts (DASA), salicylideneanilines, dihydropyrenes, anthracene dimers, fulgides, diarylethenes, phenoxy naphthacenequinones, stilbenes, and the like.

The photochromic article may include a substrate selected depending on the type of the photochromic article. Examples of the substrate may include plastic lens substrate or glass lens substrate for spectacle lens substrate. For example, the glass lens substrate may be a lens substrate made of inorganic glass. Examples of a plastic lens substrate may include a styrene resin, including a (meth)acrylate resin, a polycarbonate resin, allylic resins such as a diethylene glycol bis(allyl carbonate) resin (CR-39), a vinyl resin, a polyester resin, a polyether resin, a urethane resin obtained by a reaction between an isocyanate compound and a hydroxy compounds such as diethylene glycol, a thiourethane resin obtained by a reaction between an isocyanate compound and a polythiol compound, a cured product (which is generally called a transparent resin) of a curable composition containing a (thio)epoxy compound having at least one disulfide bond in a molecule, and the like. As a lens substrate, an undyed substrate (colorless lens) may be used, or a dyed substrate (dyed lens) may be used. For example, the refractive index of the lens substrate may be around 1.60 to 1.75. However, the refractive index of the lens substrate is not limited to the above range and may be within the range or may be over or below the range. The refractive index shall herein mean a refractive index for the light of wavelength 500 nm. The lens substrate may be a lens with refractive power (so-called prescription lens) or may be a lens without refractive power (so-called non-prescription lens).

For example, a substrate can be prepared as a curable product of a curable composition containing at least one photochromic compound represented by the general formula (1) and at least one polymerizable compound by molding the curable composition in a known molding method. The curable composition may further contain at least one other photochromic compound, for example, as those listed earlier. Curing treatment may be irradiation with light and/or heat treatment. A polymerizable compound is a compound with a polymerizable group, and a curable composition can be cured as the progress of the polymerization reaction of the polymerizable compound to form a cured product. The curable composition may contain one or more additives (for example, a polymerization initiator).

The spectacle lens may be various lenses such as a unifocal lens, a multifocal lens, and a progressive power lens. The lens type is determined depending on the surface shapes of both sides of the lens substrate. Also, the lens substrate surface may be any of convex, concave, or flat. A normal lens substrate and spectacle lens have a convex object-side surface and a concave eyeball-side surface. However, the lens substrate and the spectacle lens are not limited to these. The photochromic layer may be normally disposed on the surface of the object side of the lens substrate but may be disposed on the surface of the eyeball side.

The photochromic layer may be a layer disposed directly on the surface of the substrate or may be disposed indirectly on the surface via at least one other layer. For example, the photochromic layer may be a cured layer obtained by curing a curable composition containing a photochromic compound represented by the general formula (1). The curable composition may further contain at least one other photochromic compound, for example, as those listed earlier. For example, a cured layer containing the photochromic compound can be formed by directly applying the curable composition to the surface of a substrate or applying the curable composition on a layer provided on a substrate and subsequently subjecting the applied curable composition to curing treatment. For a coating method, known coating methods such as spin coating and dip coating may be adopted. Curing treatment may be irradiation with light and/or heat treatment. The curable composition may contain a polymerizable compound and may further contain one or more additives (for example, a polymerization initiator). The curable composition can be cured as the progress of the polymerization reaction of the polymerizable compound to form a cured layer.

Liquid Crystal Compound

The above photochromic article may include at least one photochromic compound represented by the general formula (1) and at least one liquid crystal compound in a photochromic layer disposed on a substrate in one embodiment, and the photochromic article may further include at least one liquid crystal compound in a substrate containing at least one photochromic compound in one other embodiment. The coexistence of the photochromic compound represented by the general formula (1) with an oriented liquid crystalline compound in a photochromic layer and/or a substrate can enable the molecular orientation direction of the photochromic compound to be aligned in a single direction due to the oriented liquid crystalline compound. Thereby, the photochromic compound represented by the general formula (1) is

expected to develop dichroism. If the photochromic compound represented by the general formula (1) can develop dichroism, a photochromic article including the photochromic compound represented by the general formula (1) can show polarization performance in addition to photochromic performance, and, therefore, can function as a polarization article. The polarization product can exhibit a shading function and, for example, can be used as lenses for sunglasses to protect the eyes. The photochromic compound represented by the general formula (1) can be a photochromic compound that shows less performance degradation under the coexistence with a liquid crystalline compound. An example of such a performance is fading speed after irradiation with light.

The liquid crystalline compound that can be contained in the above photochromic article may be any liquid crystalline compound that can regulate the orientation direction of molecules of the photochromic compound represented by the general formula (1) in an oriented state, and at least one publicly known liquid crystal compound can be used at any amount. For example, the liquid crystalline compound can be aligned in a single direction by an alignment film with grooves on the surface. A publicly known alignment film can be used as such an alignment film.

Polymerizable Compound

In the present invention and the present description, a polymerizable compound shall refer to a compound with one or more polymerizable group in one molecule, and a "polymerizable group" shall refer to a reactive group that can undergo polymerization reaction. Examples of the polymerizable group may include an acryloyl group, a methacryloyl group, a vinyl group, a vinyl ether group, an epoxy group, a thiol group, an oxetane group, a hydroxy group, a carboxy group, an amino group, an isocyanate group, and the like.

As examples of polymerizable compounds available for forming the above substrate and the above photochromic layer, the following compounds may be exemplified.

Episulfide-based Compound

An episulfide-based compound is a compound with two or more episulfide groups in one molecule. An episulfide group is a polymerizable group that can undergo ring-opening polymerization. Specific examples of episulfide compounds may include bis(1,2-epithioethyl) sulfide, bis(1,2-epithioethyl) disulfide, bis(2,3-epithiopropyl) sulfide, bis(2,3-epithiopropylthio)methane, bis(2,3-epithiopropyl) disulfide, bis(2,3-epithiopropyldithio)methane, bis(2,3-epithiopropyldithio)ethane, bis(6,7-epithio-3,4-dithiaheptyl) sulfide, bis(6,7-epithio-3,4-dithiaheptyl) disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropy)dithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, 2-[1,1-bis(2,3-epithiopropyldithiomethylthio)methyl]-1,3-dithietane, and the like.

Thietanyl Compound

The thietanyl compound is a thietane compound having two or more thietanyl groups in one molecule. A thietanyl group is a polymerizable group that can undergo ring-opening polymerization. Some thietanyl compounds have an episulfide group together with a plurality of thietanyl groups. Such compounds are listed in the examples of the episulfide compounds described above. Other thietanyl compounds include metal-containing thietane compounds containing metal atoms in a molecule and nonmetal-containing thietane compounds containing no metal.

Specific examples of the non-metal thietane compounds may include bis(3-thietanyl) disulfide, bis(3-thietanyl) sulfide, bis(3-thietanyl) trisulfide, bis(3-thietanyl) tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2 ,5-bis[[2-(3-thietanylthio)ethyl]thiomethyl]-1,4-dithiane, 2,5-bis(3-thietanyithiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, 1,1,2,2-tetrakis(3-thietanyldithiomethylthio)ethane, and the like.

Examples of the metal-containing thietane compounds may include compounds containing, in a molecule, group 14 atoms such as a Sn atom, a Si atom, a Ge atom, and a Pb atom, group 4 atoms such as a Zr atom and a Ti atom, group 13 atoms such as an Al atom, group 12 atoms such a Zn atom, and the like, as metal atoms. Specific examples may include alkylthio(thietanylthio)tins, bis(alkylthio)bis(thietanylthio)tins, alkylthio(alkylthio)bis(thietanylthio)tins, bis(thietanylthio) cyclic dithiotin compounds, alkyl(thietanylthio)tin compounds, and the like.

Specific examples of alkylthio(thietanylthio)tins may include methylthiotris(thietanylthio)tin, ethylthiotris(thietanylthio)tin, propylthiotris(thietanylthio)tin, isopropylthiotris(thietanylthio)tin, and the like.

Specific examples of bis(alkylthio)bis(thietanylthio)tins may include bis(methylthio)bis(thietanylthio)tin, bis(ethylthio)bis(thietanylthio)tin, bis(propyithio)bis(thietanylthio)tin, bis(isopropylthio)bis(thietanylthio)tin, and the like.

Specific examples of alkylthio(alkylthio)bis(thietanylthio)tins may include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, isopropylthio(propylthio)bis(thietanylthio)tin, and the like.

Specific example of the bis(thietanylthio) cyclic dithiotin compounds may include bis(thietanylthio)dithiastannetane, bis(thietanylthio)dithiastannolane, bis(thietanylthio)dithiastanninane, bis(thietanylthio)trithiastannocane, and the like.

Specific examples of the alkyl(thietanylthio)tin compounds may include methyltris(thietanylthio)tin, dimethylbis(thietanylthio)tin, butyltris(thietanylthio)tin, tetrakis(thietanylthio)tin, tetrakis(thietanylthio)germanium, tris(thietanylthio)bismuth, and the like.

Polyamine Compound

A polyamine compound is a compound having two or more $NH_2$ groups in one molecule, can form a urea linkage by reaction with a polyisocyanate, and can form a thiourea linkage by reaction with a polyisothiocyanate. Specific examples of the polyamine compound may include ethylenediamine, hexamethylenediamine, isophoronediamine, nonamethylenediamine, undecamethylenediamine, dodecamethylenediamine, meta-xylenediamine, 1,3-propanediamine, putrescine, 2-(2-aminoethylamino)ethanol, diethylenetriamine, p-phenylenediamine, m-phenylenediamine, melamine, 1,3,5-benzenetriamine, and the like.

Epoxy-based Compound

An epoxy-based compound is a compound with an epoxy group in a molecule. An epoxy group is a polymerizable group that can undergo ring-opening polymerization. Generally, epoxy-based compounds are classified into aliphatic epoxy compounds, alicyclic epoxy compounds, and aromatic epoxy compounds.

Specific examples of the aliphatic epoxy compounds may include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentylglycol diglycidyl ether, trimethylolpropane triglycidyl ether, glycerol triglycidyl ether, diglycerol tetraglycidyl ether, pentaerythritol tetraglycidyl ether, a triglycidyl ether of tris(2-hydroxyethyl) isocyanurate, and the like.

Specific examples of the alicyclic epoxy compounds may include isophorone diol diglycidyl ether, bis-2,2-hydroxycyclohexylpropane diglycidyl ether, and the like.

Specific examples of the aromatic epoxy compounds may include resorcindiglycidylether, bisphenol A diglycidyl ether, bisphenol F diglycidyl ether, bisphenol S diglycidyl ether, diglycidyl orthophthalate, phenolic novolac polyglycidyl ether, cresol novolac polyglycidyl ether, and the like.

Besides the above, an epoxy-based compound with a sulfur atom together with an epoxy group in a molecule can be used. Such sulfur atom-containing epoxy-based compounds include chain aliphatic types and cyclic aliphatic types.

Specific examples of chain aliphatic sulfur atom-containing epoxy-based compound may include bis(2,3-epoxypropyl) sulfide, bis(2,3-epoxypropyl) disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2 ,2-bis(2,3-epoxypropylthio)-1,3-bis(2,3-epoxypropylthiomethyl)propane, 2,2-bis(2,3-epoxypropylthiomethyl)-1-(2,3-epoxy propylthio)butane, and the like.

Specific examples of the cyclic aliphatic sulfur atom-containing epoxy-based compound may include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, 2,5-bis(2,3-epoxypropylthiomethyl)-2,5-dimethyl-1,4-dithiane, and the like.

Compound with Radically Polymerizable Group

A radically polymerizable group is a group being capable of radical polymerization. Examples of the radically polymerizable groups may include an acryloyl group, a methacryloyl group, an allyl group, a vinyl group, and the like.

In the following, a compound with one or more polymerizable groups selected from the group consisting of an acryloyl group and a methacryloyl group is referred to as a "(meth)acrylate compound". Specific examples of the (meth)acrylate compounds may include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxyethoxyphenyl)propane, 2,2-bis(4-(meth)acryloxydiethoxyphenyl)propane, 2,2-bis(4-(meth)acryloxyethoxyphenyl)propane, 2,2-bis(3,5-dibromo-4-(meth)acryloyloxyethoxyphenyl)propane, 2,2-bis(4-(meth)acryloyloxydipropoxyphenyl) propane, bisphenol F di(meth)acrylate, 1,1- bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methyl thio(meth)acrylate, phenyl thio(meth)acrylate, benzyl thio(meth)acrylate, xylylenedithiol di(meth)acrylate, mercaptomethyl sulfide di(meth)acrylate, bifunctional urethane (meth)acrylates, and the like.

Specific examples of the compounds with one or more allyl groups (allyl compounds) may include allyl glycidyl ether, diallyl phthalate, diallyl terephthalate, diallyl isophthalate, diallyl carbonate, diethylene glycol bis(allyl carbonate), methoxypolyethylene glycol allyl ether, polyethylene glycol allyl ether, methoxypolyethylene glycol-polypropylene glycol allyl ether, butoxypolyethylene glycol-polypropylene glycol allyl ether, methacryloyloxypolyethylene glycol-polypropylene glycol allyl ether, phenoxypolyethylene glycol allyl ether, methacryloyloxypolyethylene glycol allyl ether, and the like.

Examples of the compounds with one or more vinyl groups, (vinyl compounds) may include $\alpha$-methylstyrene, $\alpha$-methylstyrene dimer, styrene, chlorostyrene, methylstyrene, bromostyrene, dibromostyrene, divinylbenzene, 3,9-divinylspirobi(m-dioxane), and the like.

The above photochromic article may include, in any position, at least one layer known as a functional layer for optical articles, such as a protective layer for improving durability, an anti-reflective layer, a water-repellent or hydrophilic antifouling layer, an anti-fogging layer, a primer layer for improving interlaminar adhesion.

An embodiment of the photochromic article is an optical article, and examples of the optical article include spectacle lenses, lenses for goggles, visor (sunshade) parts of sun visors, shield members of helmets, and the like. For example, an optical article with anti-glare functions can be obtained by coating the curable composition on a substrate for these optical articles and subjecting the coated composition to curing treatment to form a photochromic layer.

Eyeglasses

One aspect of the present invention relates to eyeglasses with spectacle lenses, which is one embodiment of the photochromic article. More detailed information about the spectacle lenses of these eyeglasses was as described earlier. The eyeglasses provided with such spectacle lenses can exhibit an anti-glare effect outdoors, like sunglasses, by the photochromic compound being irradiated with the sunlight and developing a color, and in contrast, can recover the transparency when a wearer goes back indoors by the photochromic compound fading the color. A known technique can be applied to the structure of frames or the like of the above eyeglasses.

EXAMPLES

The present invention will be further described with reference to Examples.

In the following, at least one of a nuclear magnetic resonance (NMR) apparatus, an infrared spectrometer (IR), and a melting point measurement was used for identification of molecular structures.

Example 1

Synthesis of Compound 1

A compound 1

((E)-3-((1r,3r,5R,7S)-adamantane-2-ylidene)-4-(cyclopropyl(thiophene-3-yl)methylene) dihydrofuran-2,5-dione) was synthesized according to the following synthetic scheme.

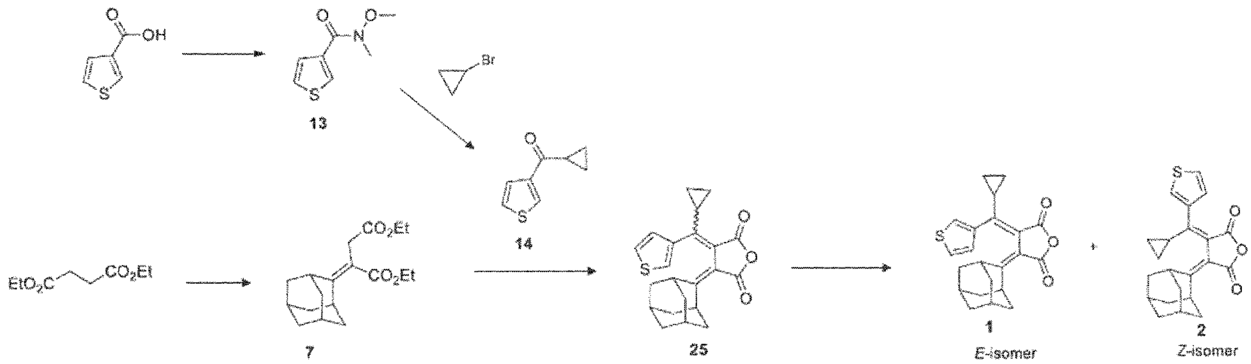

13, 14, 7, 25, 1 *E*-isomer, 2 *Z*-isomer

A compound 7 was synthesized according to the above synthetic scheme by a method disclosed in C. J. Thomas, M. A. Wolak, R. R. Birge and W. J. Lees. J. Org. Chem., 2001, 66, 1914-1918. Specifically, the compound 7 (diethyl 2-((1r,3r,5R,7S)-adamantane-2-ylidene)succinate) was synthesized by the following methods.

Diethyl succinate (19.0 mL, 114 mmol) was added to a solution of potassium tert-butoxide (12.90 g, 115.0 mmol) in tert-butyl alcohol (114 mL). After 5 min, 2-adamantanone (17.25 g, 114.8 mmol) was added and the solution stirred at reflux. After 30 min, the crude crashed out of solution, *tert*-butyl alcohol (100 mL) was additionally added and the heterogeneous mixture stirred at reflux for 22 h. Later, the crude as cooled to 0°C, 23 mL of HCl (50% w/w from 37% conc. HCl) added, the organic residue extracted with Ethyl acetate (2 $\times$ 100 mL), dried with anhydrous $Na_2SO_4$, filtered and evaporated to dryness. The resulting residue was suspended in ethanol (470 mL) and conc. HCl (37%, 9.0 mL) slowly added. After 2 days stirring at room temperature, the reaction mixture was quenched with a saturated solution of $NaHCO_3$ (100 mL). Then, the alcohol was removed under reduced pressure, the residue extracted with ethyl acetate (100 mL), washed with water (100 mL), dried with anhydrous $Na_2SO_4$, filtered and evaporated to dryness. Bulb-to-bulb distillation allowed the removal of diethyl succinate mixed with a sublimated solid impurity (140°C at 2 $\times$ $10^{-1}$ torr), giving the corresponding product as a dark red oil (21.98 g, 63%).

Compound 7: vmax (neat) 2982, 1733, 1716, 1368, 1282, 1174, 1076, 1028 $cm^{-1}$; $^1H$ NMR (400 MHz, $CDCl_3$) $\delta$H 1.09 - 1.34 (6H, m, $(CH_3)_2$), 1.67 - 1.81 (12H, m, CH), 2.69 (1H, s, CH), 3.22 (2H, s, $CH_2$), 3.54 (1H, s, CH), 3.96 - 4.04 (4H, m, $(CH_2)_2$) ppm; $^{13}C$ NMR (100 MHz, $CDCl_3$) $\delta$C 14.0, 14.1, 27.5, 34.4, 34.5, 34.9, 36.5, 38.9, 39.1, 60.0, 60.4, 114.3, 162.3, 168.3, 171.3 ppm; HRMS (ESI) found $[M+Na]^+$ = 329.1726 $C_{18}H_{26}O_4$ requires $[M+Na]^+$ = 329.1729.

A compound 13 and a compound 14 were synthesized according to the above synthetic scheme by a method disclosed in WO 2011/042918. Specifically, the compound 13 (N-methoxy-N-methylthiophene-3-carboxamide) and the compound 14 (cyclopropyl(thiophene-3-yl)methanone) were synthesized by the following methods.

Thionyl chloride (74.0 mL, 1.02 mol) was added dropwise to a solution of thiophene-3-carboxylic acid (25.01 g, 195.2 mmol) in dry dichloromethane (424 mL) at 0 °C. After stirring at room temperature for 30 min, the reaction mixture was refluxed for 4h ($CaCl_2$ drying tube fitted on top of the condenser). After cooling to room temperature, the crude was evaporated to dryness giving a brown powder. N,O-dimethylhydroxylamine hydrochloride (20.93 g, 214.6 mmol) was added to the latter, and the solids suspended in dry dichloromethane (300 mL). Triethylamine (55 mL, 395 mmol) was added dropwise to the resulting mixture at 0°C under $N_2$. After stirring at room temperature for 3h, the crude was poured into water (250 mL), the phases separated, and the aqueous phase extracted with dichloromethane (2 $\times$ 100 mL). The organic phase was washed with water (2 $\times$ 200 mL), dried with anhydrous $Na_2SO_4$, filtered and evaporated to dryness, affording the target product as an amber oil (30.15 g, 90%).

Compound 13: vmax (neat) 3114, 1608, 1515, 1420, 1350, 1217, 1185, 979, 852, 819, 724, 530 $cm^{-1}$; $^1H$ NMR (300 MHz, $CDCl_3$) $\delta$H 3.37 (3H, s, CH 3), 3.66 (3H, s, $CH_3$), 7.28 (1H, dd, J = 5.1, 3.0 Hz, Ar-H), 7.57 (1H, dd, J = 5.1, 1.2 Hz, Ar-H), 8.07 (1H, dd, J = 3.0, 1.2 Hz) ppm; $^{13}C$ NMR (77 MHz, C $DCl_3$) $\delta$C 33.3, 61.2, 124.8, 129.0, 130.8, 134.5, 163.8 ppm; HRMS (ESI) found $[M+H]^+$ = 249.9535 C7$H_8$BrNO2s requires $[M+H]^+$ = 249.9537.

Magnesium turnings (7.66 g, 0.315 mol) and a few crystals of iodine were added to a dry three-neck 1 L round-bottom flask (equipped with a water condenser) and heated with a heat gun. Then, anhydrous THF (210 mL) was added

while stirring. Afterwards, bromocyclopropane (25.0 mL, 0.312 mol) was added portion wise with occasional heating with a heat gun until the reaction was self-sustaining (reaction mixture went from orange to colourless). Once the magnesium turnings were consumed, N-methoxy-N-methylthiophene-3- carboxamide (compound 13) (21.09 g, 0.1232 mol) in anhydrous THF (214 mL) was added dropwise under $N_2$ while stirring. The reaction mixture was stirred for 2 h at room temperature under $N_2$ and then quenched with the addition of HCl (2 M) at 0 °C. Then, water (100 mL) was added, the phases separated, the aqueous phase extracted with Ethyl acetate (2 × 100 mL), the organic phase washed with water (2 × 100 mL), dried with anhydrous $Na_2SO_4$, filtered and evaporated to dryness. The resulting orange oil (20.40 g) was filtered through a plug of silica (Aldrich silica gel 60 A 230-400 mesh 40-63 μm; eluent: 10% ethyl acetate in petroleum ether to 20% ethyl acetate in petroleum ether, fraction 1) giving the target product as a yellow oil (16.88 g, 88%).

Compound 14: vmax (neat) 1653, 1510, 1418, 1230, 1169, 1035, 1005, 909, 877, 816, 799, 732, 696 $cm^{-1}$; $^1H$ NMR (400 MHz, $CDCl_3$) δH 0.98 - 1.03 (2H, m, $CH_2$), 1.16 - 1.24 (2H, m, $CH_2$), 2.49 - 2.55 (1H, m, CH), 7.33 (1H, dd, J = 5.1, 2.9 Hz, Ar-H), 7.59 (1H, dd, J = 5.1, 1.0 Hz, Ar-H), 8.13 (1H, dd, J = 2.9 Hz, 1.2 Hz) ppm; $^{13}C$ NMR (100 MHz, $CDCl_3$) δC 11.3, 18.3, 126.2, 127.0, 131.5, 143.1, 194.7 ppm.

The compound 1 was synthesized by the following method.

A solution of diethyl 2-((1r,3r,5R,7S)-adamantan-2-ylidene)pentanedioate (11.01 g, 35.93 mmol) in anhydrous toluene (50 mL) was added dropwise to a suspension of NaH [60% dispersion in mineral oil] (2.10 g, 52.5 mmol) in anhydrous toluene (134 mL) at room-temperature under N2. The suspension was heated to 60 °C, one drop of ethanol added and the mixture stirred for 2 h. Then, cyclopropyl(thiophen-3-yl)methanone (5.00 g, 32.8 mmol) in anhydrous toluene (50 mL) was added dropwise at 60°C, one drop of methanol added and the reaction mixture left stirring at 60°C for 18 h under $N_2$. The crude was cooled to room-temperature, slowly poured into ice (200 mL) while stirring, the phases separated and the organic phase extracted with water (2 × 100 mL). The aqueous phase was acidified (pH = 1 - 2) with the addition of HCl (2 M), extracted into diethylether (3 × 100 mL), the organic phase dried with anhydrous $Na_2SO_4$, filtered and evaporated to dryness, affording a yellow foam (10.91 g). The resulting residue was refluxed for 6 h in a 10% potassium hydroxide ethanolic solution (400 mL). Then, the crude was evaporated to dryness, water (250 mL) added and the solution acidified (pH = 1 - 2) with the addition of HCl (2 M). Afterwards, the residue was extracted into diethylether (3 × 100 mL), washed with water (100 mL), dried with anhydrous $Na_2SO_4$, filtered and evaporated to dryness, affording an orange foam (10.60 g) that corresponded to a mixture of E and Z diacids. Then, the solid was dissolved in acetic anhydride (60 mL) and heated at reflux for 5 min. The reaction mixture was cooled to room-temperature and the acetic anhydride rapidly evaporated off affording a brown solid (8.60 g). Flash column chromatography (Aldrich silica gel 60 A 230-400 mesh 40-63 μm; eluent: 10% Ethyl acetate in petroleum), followed by trituration with petroleum, afforded the target E isomer (compound 1) (1.85 g), Z isomer (compound 2) (0.97 g) and an E and Z mixture (2.02 g) - overall (4.84 g, 40%).

Compound 1: off-white powder; mp = 138 - 143°C; vmax (neat) 2916, 2 849, 1798, 1748, 1585, 1229, 1211, 987, 923, 897 $cm^{-1}$; $^1H$ NMR (300 MHz, C $DCl_3$) δH 0.52 - 0.57 (2H, m, CH), 0.81 - 0.87 (2H, m, CH), 0.94 - 1.01 (2H, m, CH), 1.56 - 1.91 (11H, m, CH), 2.41 (1H, s, CH), 3.17 - 3.26 (1H, m, C H), 4.09 (1H, s, CH), 6.88 - 6.90 (1H, m, Ar-H), 7.09 - 7.10 (1H, m, Ar-H), 7. 31 - 7.34 (1H, m, Ar-H) ppm; $^{13}C$ NMR (77 MHz, $CDCl_3$) δC 7.29, 7.95, 15.8, 27.0, 27.3, 33.4, 36.2, 37.8, 39.0, 39.4, 114.0, 120.9, 125.3, 125.8, 126.4, 128. 4, 136.6, 154.7, 163.2, 164.2, 171.0 ppm; HRMS (ESI) found $[M+H]^+$ = 367.135 4 $C_22H_{22}O_3S$ requires $[M+H]^+$ = 367.1368.

Evaluation on Performance under Coexistence with Oriented Liquid Crystalline Compound

The compound 1 was measured for the fading speeds in an oriented field (i.e., under coexistence with an oriented liquid crystalline compound) and a non-oriented field according to the following methods.

A test cell, prepared in the method described below, was irradiated with light through an aero mass filter using a xenon lamp for 15 minutes (900 seconds) to develop the color of the photochromic compounds in the test cell. After that, the light irradiation was stopped. The irradiation with light was conducted such that the irradiance and the tolerance of the irradiance were to be the values shown in the following Table 1 as regulated in JIS T 7333:2005.

Table 1

| Wavelength range (nm) | Irradiance ($W/m^2$) | Tolerance of irradiance ($W/m^2$) |
|---|---|---|
| 300-340 | <2.5 | - |
| 340-380 | 5.6 | ±1. 5 |
| 380-420 | 12 | ±3.0 |
| 420-460 | 12 | ±3.0 |

(continued)

| Wavelength range (nm) | Irradiance (W/m$^2$) | Tolerance of irradiance (W/m$^2$) |
|---|---|---|
| 460- 500 | 26 | ±2.6 |

The luminous transmittance T (%) of a lens before color development was standardized to 1 and the luminous transmittance after the color development was standardized to 0, and the time until the standardized transmittance reaches 0.5 after the stop of the irradiation with light was defined as T(1/2). T(1/2)s in the oriented field and the non-oriented field were calculated. A fading speed change rate was calculated from the following expression.

Fading speed change rate = T(1/2) in the oriented field / T(1/2) in the non-oriented field

Preparation of Test Cell

Oriented Field

1% by mass of the compound 1 was added to 100% by mass of a host liquid crystal and fully dissolved under stirring for 30 minutes at a temperature range of room temperature to 50°C. After the temperature of the liquid crystalline mixture was fallen to room temperature, the liquid crystalline was filled in a test cell utilizing capillary pressure. The test cell is constituted by a pair of glass plates. Two glass plates constituting the test cell are each provided with, inside the cell, a polyimide that is rubbing-processed in one direction and formed with plural numbers of streaky grooves on the surface. In the test cell, two glass plates were faced so that the rubbing direction of the polyimide films faced the same direction. The cell gap was 40 μm. The polyimide film functioning as an orientation film can make the host liquid crystal uniaxially orientated. For the host liquid crystal, 4-cyano-4'-pentylbiphenyl, which indicates a liquid crystalline phase at room temperature (23°C), was used.

Non-Oriented Field

A test cell of the non-oriented field was prepared by the method described about the preparation of the test cell of the oriented field, except that 4-cyano-3-fluorophenyl 4-butylbenzoate, which indicates an isotropic phase at room temperature (23°C), was used as the host liquid crystal, and a test cell that was not provided with the polyimide film described above was used as the test cell.

Comparative Examples 1 and 2

Evaluation was made according to the method stated about Example 1, except that the following comparative compounds 1 and 2 were used instead of the compound 1.

(Comparative compound 1)

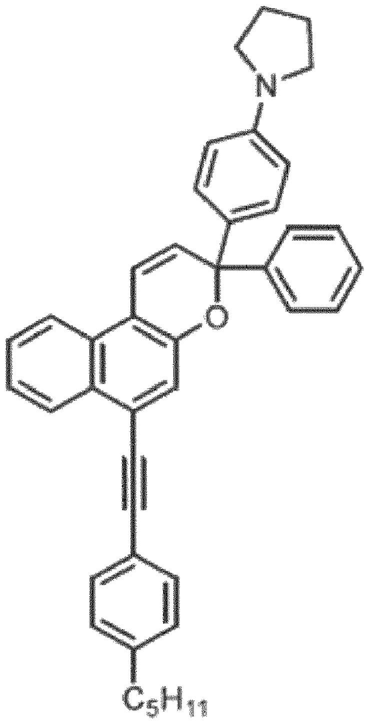

[0093] (Comparative compound 2)

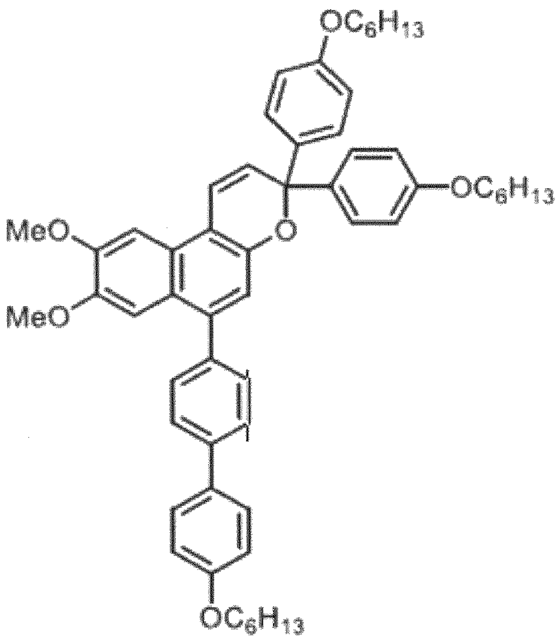

Table 2 shows the above results.

Table 2

| | Compound | Fading speed change rate |
|---|---|---|
| Example 1 | Compound 1 | 1.1 |
| Comparative Example 1 | Comparative Compound 1 | 1.6 |
| Comparative Example 2 | Comparative compound 2 | 2.4 |

A comparison between the evaluation result of Example 1 and the evaluation results of Comparative Examples 1 and 2 in Table 2 reveals that the compound represented by the general formula (1) shows less performance deterioration under the coexistence with an oriented liquid crystal compound (more specifically, change of the fading speed is small).

Confirmation of Photochromic Performance (Dynamic Range)

The test cell of the oriented field and the test cell of the non-oriented field in Example 1 were each measured for luminous transmittance (Tini) in an unirradiated state and luminous transmittance (Tact) at the time when the color development was saturated under the irradiation with artificial sunlight for a certain period of time using an ultraviolet and visible spectrophotometer (UV-1900i, manufactured by Shimadzu Corporation, the measurement wavelength: 800 to 250 nm, measured at a pitch of 2 nm wavelength, fast mode). The measurement was conducted at room temperature (23°C). The dynamic range "(Tini) - (Tact)" can be used as an index of the degree of the color development of a colored body to a colorless body, and it is considered that the larger the value of the dynamic range is, the better photochromic performance is.

Confirmation of Polarization Performance (Dichroic Ratio, Polarization Degree)

The test cell of the oriented field in Example 1 was measured for an absorbance (Apara) and a luminous transmittance (Tpara) when polarized light parallel with the rubbing direction (i.e., the orientation direction of the liquid crystalline compound) of the polyimide film is incident, and an absorbance (Aperp) and a luminous transmittance (Tperp) when polarized light is incident in a direction perpendicular to the rubbing direction. The dichroic ratio R was calculated by the following expression 1, and the polarization degree P was calculated by the following expression 2.

$$\text{Expression 1: } R = Apara/Aperp$$

$$\text{Expression 2: } P = (Tperp - Tpara)/(Tperp + Tpara) * 100$$

Table 3 shows the above results.

Table 3

| | Non-Oriented Field | Oriented Field |
|---|---|---|
| Dynamic range | 3.2 | 5.8 |
| Dichroic ratio R | | 1.9 |
| Polarization degree P | | 4.7 |

The results listed in table 3 reveal that the compound 1 evaluated in Example 1 shows better photochromic performance in an oriented field than in a non-oriented field and exhibits polarization performance because the compound 1 shows dichroism under the coexistence with an oriented liquid crystal compound.

Finally, the aspects described above are summarized as follows.

According to one aspect, a photochromic compound represented by the general formula (1) is provided.

The photochromic compound represented by the general formula (1) can be a photochromic compound that shows less performance degradation under the coexistence with an oriented liquid crystalline compound.

In one embodiment, X in the general formula (1) may denote an oxygen atom.

According to one aspect, a photochromic article including the above photochromic compound is provided.

In one embodiment, the photochromic article may at least have a substrate and a photochromic layer containing the photochromic compound described above.

In one embodiment, the photochromic layer may further contain a liquid crystal compound.

In one embodiment, the photochromic article can be a spectacle lens.

In one embodiment, the photochromic article can be a goggle lens.

In one embodiment, the photochromic article can be a visor part of a sun visor.

In one embodiment, the photochromic article can be a shield member of a helmet.

According to one aspect, eyeglasses with the spectacle lens described above are provided.

Various aspects and embodiments described in the present description can be combined as any combination

of two or more of these.

The embodiments disclosed here are to be understood as examples and do not restrict the scope of the invention in the entire points. The scope of the invention is determined not by the above description but by the claims and is intended to include the interpretations that are equivalent to the claims and all modifications within the scope of claims.

One aspect of the present invention can be useful in the technical fields of eyeglasses, goggles, sun visors, helmets, and the like.

**Claims**

**1.** A photochromic compound represented by the following general formula (1):

General formula (1)

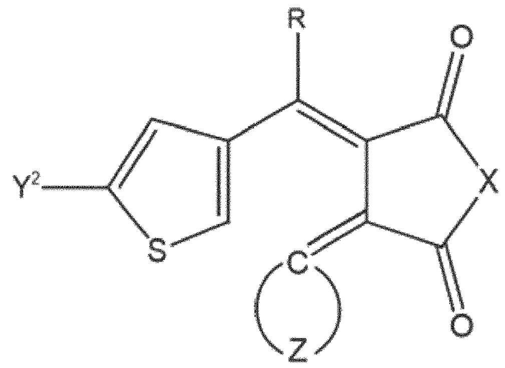

wherein, in the general formula (1),

X denotes an oxygen atom or a nitrogen atom unsubstituted or substituted by a substituent selected from the following $Y^1$ group:
$Y^1$ group: $-R^1$, $-A^1(B^1)_l(A^2)_m(B^2)_nR^2$, $-A^3A^4$, $-A^5R^3$
$R^1$ denotes a cyano group, or an alkyl group or an aryl group which may each be substituted,
$R^2$ denotes an alkyl group, a naphthyl group, or a naphthylalkyl group which may each be substituted,
$R^3$ denotes a halogen atom, a cyano group, or a nitro group,
$A^1$, $A^2$, $A^3$, and $A^5$ each independently denote an alkylene group, an alkylidene group, a cycloalkylene group, or an alkylcycloalkane-diyl group which may each be substituted,
$A^4$ denotes a naphthyl group which may be substituted,
$B^1$ and $B^2$ each independently denote any one of the divalent groups selected from the following group:

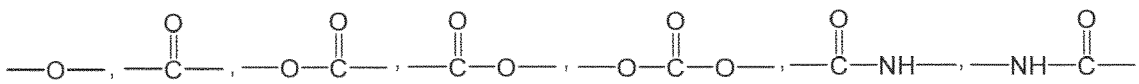

l, m, and n are each independently 0 or 1, provided that n is 0 if m is 0,
$Y^2$ denotes a hydrogen atom, or a substituent selected from the above $Y^1$ group, R denotes a hydrogen atom, a trifluoromethyl group, or a cyclopropyl group which may be substituted,

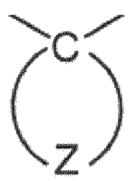

denotes a norbornylidene group, a bicyclo[3.3.1]nonylidene group, or an adamantylidene group which may each be substituted.

**2.** The photochromic compound according to claim 1, wherein X in the general formula (1) denotes an oxygen atom.

**3.** A photochromic article comprising the photochromic compound according to claim 1 or 2.

**4.** The photochromic article according to claim 3, which comprises at least:

a substrate; and
a photochromic layer containing the photochromic compound.

**5.** The photochromic article according to claim 4, wherein the photochromic layer further comprises a liquid crystal compound.

**6.** The photochromic article according to any of claims 3 to 5, which is a spectacle lens.

**7.** The photochromic article according to any of claims 3 to 5, which is a goggle lens.

**8.** The photochromic article according to any of claims 3 to 5, which Is a visor part of a sun visor.

**9.** The photochromic article according to any of claims 3 to 5, which is a shield member of a helmet.

**10.** Eyeglasses comprising the spectacle lens according to claim 6.

Europäisches Patentamt
European Patent Office
Office européen des brevets

# EUROPEAN SEARCH REPORT

Application Number
EP 22 16 6176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H07 285931 A (TOKUYAMA CORP) 31 October 1995 (1995-10-31) * the whole document * ----- | 1-10 | INV. C07D409/06 C09K9/00 C09K11/00 |
| X | EP 0 629 626 A2 (TOKUYAMA CORP [JP]) 21 December 1994 (1994-12-21) * the whole document * ----- | 1-10 | |
| X | JP H06 345772 A (TOKUYAMA SODA KK) 20 December 1994 (1994-12-20) * the whole document * ----- | 1-10 | |
| X | EP 0 696 582 A1 (TOKUYAMA CORP [JP]) 14 February 1996 (1996-02-14) * the whole document * ----- | 1-10 | |

TECHNICAL FIELDS SEARCHED (IPC)

C07D
H05B
C09K

The present search report has been drawn up for all claims

1

| Place of search | Date of completion of the search | Examiner |
|---|---|---|
| Munich | 18 May 2022 | Sarakinos, Georgios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 16 6176

This annex lists the patent family members relating to the patent documents cited in the above-mentioned European search report. The members are as contained in the European Patent Office EDP file on
The European Patent Office is in no way liable for these particulars which are merely given for the purpose of information.

18-05-2022

| Patent document cited in search report | | Publication date | Patent family member(s) | | | Publication date |
|---|---|---|---|---|---|---|
| JP H07285931 | A | 31-10-1995 | NONE | | | |
| EP 0629626 | A2 | 21-12-1994 | AU | 679513 | B2 | 03-07-1997 |
| | | | DE | 69421322 | T2 | 27-07-2000 |
| | | | EP | 0629626 | A2 | 21-12-1994 |
| | | | ES | 2140506 | T3 | 01-03-2000 |
| | | | JP | 3138117 | B2 | 26-02-2001 |
| | | | JP | H072824 | A | 06-01-1995 |
| | | | US | 5708063 | A | 13-01-1998 |
| JP H06345772 | A | 20-12-1994 | NONE | | | |
| EP 0696582 | A1 | 14-02-1996 | AU | 688531 | B2 | 12-03-1998 |
| | | | DE | 69515758 | T2 | 10-08-2000 |
| | | | EP | 0696582 | A1 | 14-02-1996 |
| | | | ES | 2145220 | T3 | 01-07-2000 |
| | | | US | 5631382 | A | 20-05-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5645767 A
- JP H072824 B
- WO 2011042918 A


### Non-patent literature cited in the description


- **C. J. THOMAS ; M. A. WOLAK ; R. R. BIRGE ; W. J. LEES.** *J. Org. Chem.,* 2001, vol. 66, 1914-1918